# EUROPEAN PATENT SPECIFICATION

(11) **EP 1 722 699 B1**
(45) Date of publication and mention of the grant of the patent: **23.10.2013**
(21) Application number: 04785834.5
(22) Date of filing: 25.02.2004
(51) Int. Cl.: A61B 17/50

(54) **APPARATUS FOR ACOUSTIC/MECHANICAL TREATMENT OF EARLY STAGE ACNE**
GERÄT FÜR DIE AKUSTISCHE/MECHANISCHE BEHANDLUNG VON AKNE IM FRÜHSTADIUM
DISPOSITIF DE TRAITEMENT ACOUSTIQUE/MECANIQUE DE L'ACNE PRECOCE

(43) Date of publication of application: 22.11.2006
(60) Divisional application: 12199356.2
(73) Proprietor: L'Oréal SA, 75008 Paris (FR)
(72) Inventor: PILCHER, Kenneth A., Seattle, WA 98107 (US); GIULIANI, David, Mercer Island, WA 98040 (US); MEGINNISS, Stephen M., Seattle, WA 98119 (US)
(74) Representative: Khairallah, Murielle
(86) International application number: PCT/US2004/000533
(87) International publication number: WO 2005/091748

(56) References cited:
- EP-A1- 0 650 375
- WO-A1-99/49915
- WO-A1-03/096961
- US-A- 2 076 410
- US-A- 2 338 339
- US-A- 4 203 431
- US-A- 5 624 416
- US-A- 5 687 442
- US-B1- 6 245 093

## Description

### Technical Field

This invention relates generally to treatment of early stage acne, and more particularly concerns mechanical and/or acoustic devices for applying energy to the skin in the vicinity of the early stage acne lesion, i.e. the sebaceous plug.

### Background of the Invention

Common acne, known more specifically as acne vulgaris, is generally regarded to be the most treated skin condition in the United States. Prompt and appropriate treatment of acne, particularly in its early stages, is important for both resolving the early stage condition and preventing more severe acne conditions, which have possible permanent effects, including the possibility of severe scarring. While acne can occur in men and women of all ages, it typically occurs in adolescents and young adults.

The earliest evidence of acne is the formation of a sebaceous plug. The sebaceous plug, which is formed in the individual skin pores (follicles), is typically not visible to the unassisted eye, but can be seen under a microscope or other optical lens device. It is formed when a combination of corneocytes and sebum, which are both natural components of the skin, block the pore opening, and specific colonies of bacteria within the skin pore then expand in numbers. The plug of cells and sebum may adhere to the wall of the skin pore, leading to material aggregation in the pore, and subsequent widening of the pore. This situation may in turn result in the further accumulation of sebum and other cellular material, and the eventual possible rupture of the follicular wall, followed by an inflammatory response and the subsequent formation of inflamed papules and inflamed pustules, typically referred to as pimples.

Existing systemic treatments of acne include oral antibiotics, retinoids and hormonal treatments. Each of these treatments, while effective to various extents, has its own significant side effects and disadvantages. For instance, oral antibiotic treatment reduces the number of bacteria in the skin pores, but does not decrease the rate of sebum secretions or the actual number of the sebaceous plugs formed. Disadvantages of the various treatments include various undesirable skin reactions, including skin dryness, fluid loss and possible hair loss. Typically, all such treatments irritate the skin to some extent..

### Prior Art

Prior art concerning localized treatment for acne can be classified generally as "mechanical" or "chemical."

Mechanical methods include vacuum devices, mechanized scrub brushes and manual loop-like instruments, such as shown in U.S. Patents No. 5,624,416 and 4,175,551. Use of these devices is typically site-specific and usually requires a specific technique, making them difficult to use. Methods that use heat generated by electrical resistance or ultrasound are also known, such as shown in U.S. Patent No. 6,245,093. Still other methods claim to be able to kill target micro-organisms, including those that cause acne, using selected frequencies of electrical current, such as shown in U.S. Patent No. 5,891,182.

In the beauty/skin care industry, the use of microabrasion is also a popular treatment for "rejuvenating" skin. However, this technique of removing some layers of the cornified skin layer by abrasive materials can cause intense irritation.

Chemical methods for acne, including topical and systemic treatments and their possible side effects, are listed in Tables I and II below, respectively.

**Table I. Common Topical Acne Treatments**

| **Treatment** | **Possible Side Effect** |
|---|---|
| Soaps and detergents to remove sebum from skin surface | Drying of skin surface (xerosis) if used in excess |
| Astringents and short chain alcohols to remove oily materials and water in upper epidermal areas | Drying of the skin surface and breakdown of the skin's barrier function, and eventual microbial entrance into the body |
| Antibacterial agents (e.g. benzoyl peroxide, salicylic acid), that can destroy bacteria when the agent is in immediate contact with the microorganisms | <5% penetrates into the pores, with the rest possibly interacting with corneocytes causing irritation and erythmea (red skin), and contact dermatitis |

**Table II. Common Systemic Acne Treatments**

| **Treatment** | **Possible Side Effect** |
|---|---|
| Oral antibiotics | Photosensitivity, gastrointestinal problems, and bacterial antibiotic resistance |
| Hormonal manipulations to control sebaceous gland size and secretion rate by regulating androgens and estrogens | Increased risk of thromboembolism, feminization in men, and other undesirable effects. |
| Retinoids, which likely change the cohesiveness of follicular epithelial cells | Teratogenic, and other strong negative side effects |

With the present apparatus, conditions that lead to early stage acne are prevented and early stage acne is effectively treated by maintaining or restoring the pore openings to an open state, to allow continuing exudation from the sebaceous gland, to encourage maintaining an aerobic state within the pore, and to prevent the development of more severe acne conditions, without the inconvenience, side effects and other limitations present in existing treatments.

Document EP 0 650 375 discloses a device for cleaning skin pores according to the preamble of claim 1.

### Disclosure of the Invention

Accordingly, the invention is an apparatus for the treatment of acne, comprising: at least two contacting elements having end faces which are in substantially the same plane, wherein at least one contacting element is a moving contacting element; a mounting assembly for holding the contacting elements substantially adjacent to each other; and an assembly for reciprocally moving said at least one moving contacting element relative to at least one adjacent contacting element, wherein when the apparatus is positioned so that the end faces of the contacting elements contact the skin, an action on the skin is produced to remove sebum plugs from skin pores, permitting ready removal thereof from the skin.

The contacting elements can comprise either elements of rigid material, compliant material or rows of bristle tufts. The apparatus further can be used for an effective cleansing treatment of skin which does not have an acne condition. Still further, the apparatus could comprise a single moving contact element.

Another aspect, which is not part of the invention, is a method for treatment of skin comprising the steps of: a first step of deforming the skin from a neutral position to a first deformed position at which point the skin has reached approximately its elastic limit; a second step permitting the skin to return to said neutral position; and repeating the first, second steps, within a frequency range of 20 Hz to 1 kHz, to produce an action on the skin which results in the cleansing of the skin, including removal of undesired material from skin pores. The method is effective for acne treatment as well as general skin cleansing.

### Brief Description of the Drawings

Figure 1 shows a cross-sectional view of a typical skin pore.
Figure 2 is a cross-sectional view of a comedone-plugged acroinfundibulum.
Figure 3 is a cross-sectional view of the comedone-plugged acroinfundibulum, taken along lines 3-3 in Figure 2.
Figure 4 shows strain characteristics for skin for differing degrees of applied stress.
Figures 5A and 5B show the relative displacement of a single tuft of bristles moving against the skin.
Figure 6 is an exploded view of one aspect of the mechanical acne treatment device.
Figure 7 is a schematic view of a fixed contact element part of the apparatus of Figure 6.
Figure 8 is a schematic view of a movable contact element part of the apparatus of Figure 6.
Figures 9A, 9B, 9C and 9D show the effect on a typical sebaceous plug positioned within a skin pore subjected to the action of the apparatus of Figure 6.
Figure 10 is a schematic view of a variation of the mechanical device of Figure 6.
Figure 11 is a schematic view of a fixed contact element part of the apparatus of Figure 10.
Figure 12 is a schematic view of a movable contact element part of the apparatus of Figure 10.
Figures 13A, 13B, 13C and 13D show the effect on a sebaceous plug positioned in a skin pore subjected to the action of the apparatus of Figures 10-12.
Figure 14 shows a variation of the mechanical device shown in Figure 7, wherein a compliant material is applied to the contact element surface.
Figure 15, is a diagram showing an alternate embodiment of the present invention with alternately linearly movable rows of bristle tufts, surrounded by a circular row of fixed bristle tufts.
Figure 16 is a top view of the device shown in Figure 15.
Figure 17 is a diagram showing a variation of the mechanical device shown in Figure 15, in which one set of bristle tufts is fixed, and the other set moves linearly.
Figure 18 is a top view of the device shown in Figure 17.
Figure 19 is a diagram showing a further alternate embodiment of the present invention with alternately rotationally movable sets of bristle tufts.
Figure 20 is a top view of the mechanical device shown in Figure 19.
Figure 21 is a diagram showing a variation of the mechanical device shown in Figure 16 with a single set (two rows) of rotationally moving bristle tufts.
Figure 22 is a top view of the mechanical device shown in Figure 21.
Figure 23 is a schematic block diagram showing the control means for controlling the amplitude of motion as a function of pressure applied to the skin.
Figure 24 is a schematic representation of the human face showing the various regions of the face that differ in the degree of sebum secretion and incidence of acne lesions.
Figure 25 is a block diagram of a timer structure useful with the present invention.

### Best Mode for Carrying Out the Invention

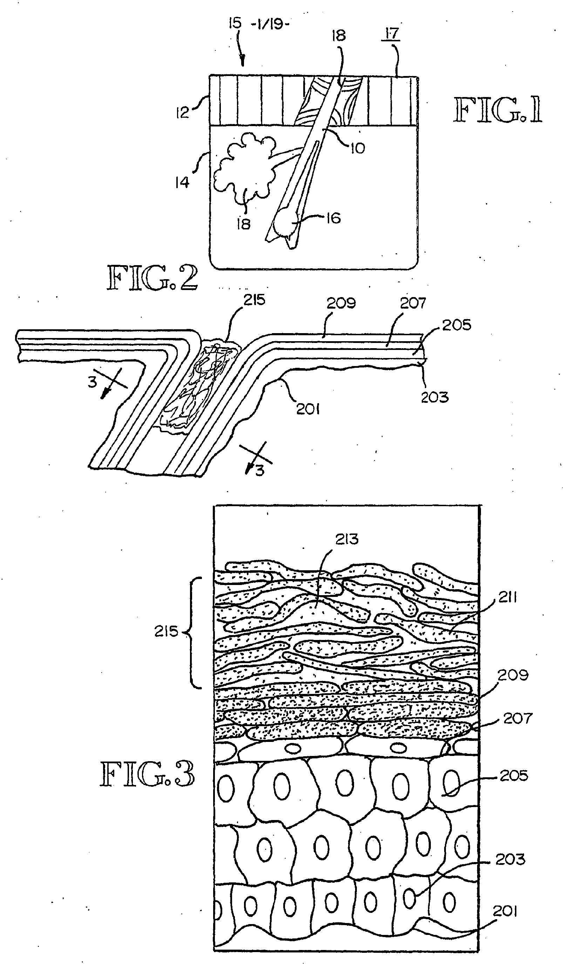

Figure 1 is a representation of a typical skin pore, including the epidermis and dermis layers of the skin. The skin pore 10, also referred to as a follicle, includes a normal hair 16 and an associated sebaceous gland 18. The sebaceous gland 18 normally produces sebum lipids. The production of sebum, however, is typically not sufficient alone to result in acne. Further, acne lesions do not occur if the sebum lipids are free to reach the surface of the skin. However, when the skin pore or follicle becomes blocked, such as by an overproduction of corneocytes, or inadequate shedding of the corneocytes (as shown in Figure 2), the balance of the skin system is upset. The blocked follicles lead to the formation of closed, but non-inflamed, sebaceous plugs. The sebaceous plug (microcomedone) 215 shown in vertical cross-section in Figure 2 and in horizontal cross-section in Figure 3 forms in the acroinfundibulum portion of the follicle, which is the upper portion of the follicle.

Following the initial formation of the sebaceous plug, if the pH and oxygen tension are within certain ranges below the closed sebaceous plug, the number of *Propionibacteria* acnes bacteria expands, leading to a pathogenic condition. This leads further to a sequence of actions and reactions within the follicle, including damage to the follicular wall, comprising skin layers 201, 203, 205, 207 and 209, and extrusion of accumulated materials into the dermis portion of the skin, resulting in an inflammatory response which leads to skin lesions and pustules.

In the present invention, the focus is on maintaining the acroinfundibulum portion of the follicle in an open state, which eliminates the environment in which the acne bacteria can thrive within the follicle, and encourages establishing an aerobic state within the follicle, while at the same time minimizing the amount of sebum that can accumulate within the infundibulum portion of the follicle.

The basic approach of the present invention is to reopen the individual pores that may have been blocked by the plug of corneocytes 211 and sebum lipids 213 (Figure 3). It is based on the discovery that application of differential motion locally to the pore opening will open a blocked pore. The opening of the pore is due to the fact that the blocking materials within the follicles have different physical properties than the wall of the infundibulum and the surrounding skin. With the present invention, the skin area is deformed slightly and then released to a relaxed position and then deformed slightly in the opposite direction and then again released to a relaxed position, at a specified frequency, which results in the plugs being loosened from their position in the skin pores. The loosened plugs can then be readily removed, such as by wiping or washing, permitting thereafter normal skin secretion of lipids, and consequently avoiding the consequences of more fully developed acne.

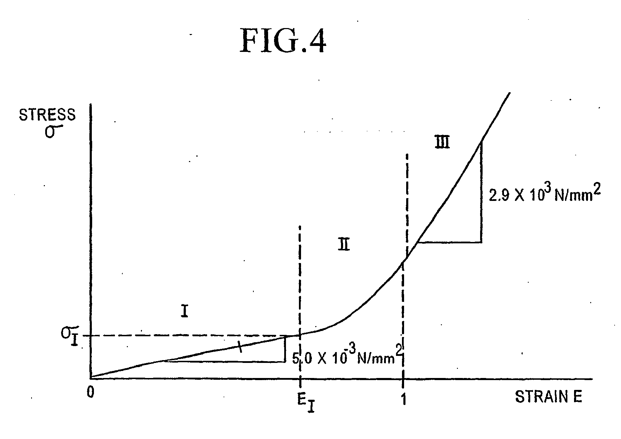

Figure 4 shows three regions of the skin's elastic modulus, *i*.*e*. the amount force (stress) required to deform the skin a given degree (strain). This curve is the result of the unique mechanical organization of the skin. This mechanical organization can be thought of as large numbers of loose collagen fibers connected together at randomly distributed nodal points. The mechanical behavior of such a system is very similar to that of a woven material such as a nylon stocking. As the material is stretched, the fibers are first straightened out and become oriented in the direction of the stress (shown in Figure 4 region I). A relatively small amount of stress is required to produce this level of strain, with a modulus of elasticity typically 5 x 10⁻³ N/mm². Skin is elastic over this range.

Generally at the end of region I and slightly into region II, the elasticity of the skin substantially decreases and the skin becomes taut. In region II, some fibers become fully aligned in the direction of the stress and then carry stress directly. Further deformation will result in ever-increasing numbers of collagen fibers being recruited to support the stress. The modulus of elasticity, or stiffness, of the skin increases rapidly as this process continues until it matches the stiffness of the collagen fibers themselves (region III). The modulus of elasticity in this region is typically 3 x 10³ N/mm².

In the present invention, the desired differential motion applied to the skin should be of high enough amplitude to create pore opening forces, but low enough to minimize stretching of collagen fibers in the skin. Deformation should be limited to the area of region I and the low strain area of region II of Figure 4, where the collagen fibers are not significantly stretched. To that end, the mechanical characteristics of the portions of the invention that contact the skin, called the contacting elements, and the amplitude of the moving contact elements must be such that the degree of stress on the skin does not exceed some value σ_{I} thus keeping the amount of strain in the skin below the value E_{I} of Figure 4.

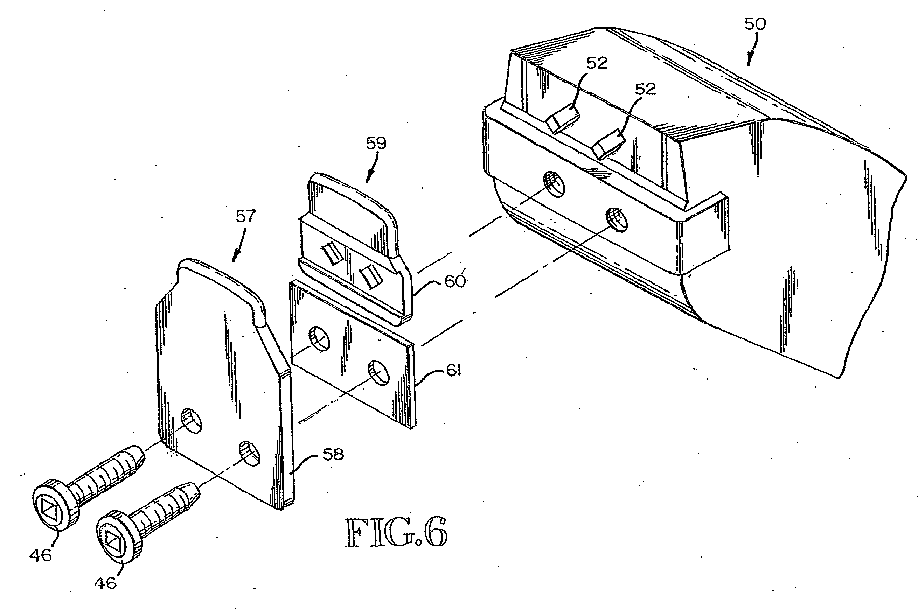

A first aspect is shown generally in Figure 6, wherein movable skin contacting elements are located in the same plane as stationary skin contacting elements, and the bi-directional differential action is shear, i.e. the two elements move in parallel to each other along their length. The arrangement shown includes two skin contact elements 57 and 59 and a backing/spacer plate 61.

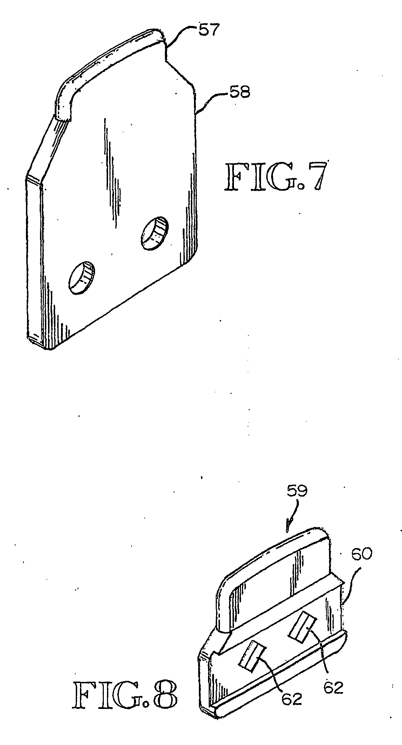

The movable and non-movable skin contacting elements are basically identical. In the aspect shown, the individual contact elements 57 and 59 are each mounted on mounting plates. The fixed contact element 57 is mounted on mounting plate 58, while the oscillating contact element 59 is mounted on mounting plate 60. The contact elements are narrow and somewhat elongated and are shown in detail in Figures 7 and 8. In the aspect shown, the dimensions of the contact elements are typically 2,03 cm (0.800 inches) long and 0,23 cm (0.09 inches) wide. The edges of each contact element are beveled; giving it a somewhat rounded end face, while the respective ends are also rounded, as shown in the drawings.

The mounting plate 58 for the fixed contact element is approximately 1.18 inches wide. The upper corners of mounting plate 58 are both cut off, at a 36° angle. The height of the mounting plate 58 is 3,56 cm (1.4 inches). Figure 8 shows the combination of contact element 59 and mounting plate 60 for the oscillating contact element. Mounting plate 60 is, in the embodiment shown, substantially rectangular, with a length of 2,54 cm (1.0 inches) and a height of 1,27 cm (0.5 inches). The upper corners of mounting plate 60 are cut off, *i.e*. beveled, at an angle of 45°, while the lower corners are rounded.

Oscillating contact element 59 is mounted on mounting plate 60 on the upper edge thereof. The upper edge of contact element 59 is approximately 0,76 cm (0.298 inches) above the upper edge of mounting plate 60. Mounting plate 60 includes two drive openings 62-62 therethrough, so that the mounting plate 60 and contact element 59 can be moved back and forth by a driver mechanism discussed below. In the embodiment shown, the drive holes 62 are approximately square, 0,40 cm (0.154 inches) on each side.

The oscillating contact element 59 on its mounting plate 60 and the fixed contact element 57 on its mounting plate 58 are then positioned immediately adjacent to each other, as shown in Figure 6. The two contact elements 57 and 59 are thus substantially in registry. The two assemblies are held together and attached to the driver mechanism 50 by two connecting screws 46-46. Hence the contact elements may be removed and replaced. The oscillating contact element assembly is movable by means of the driver that moves it reciprocally (back and forth), such that the mounting plate 60 moves parallel to mounting plate 58, and contact element 59 moves reciprocally in parallel with contact element 57.

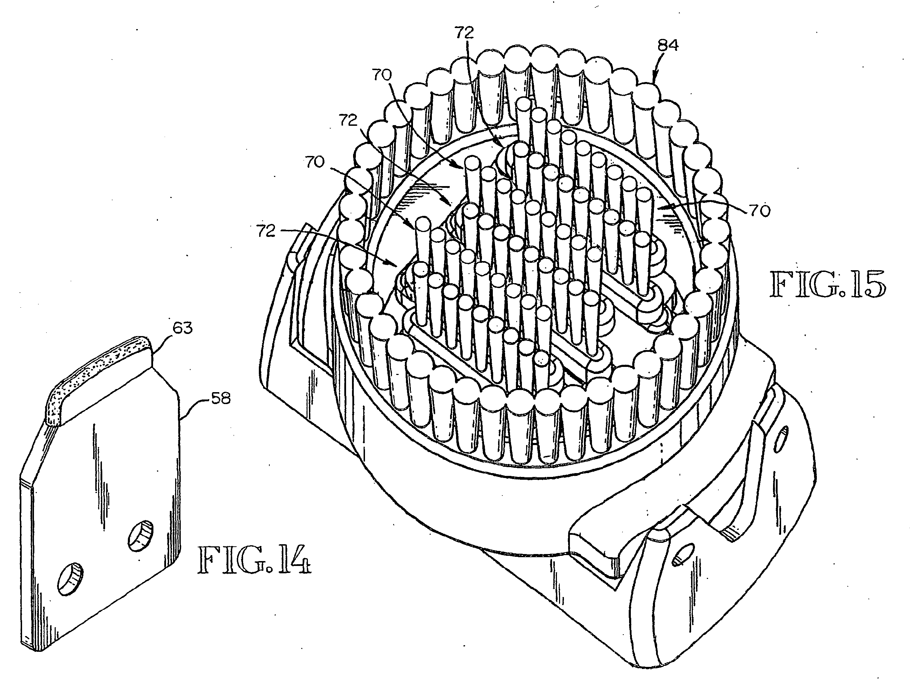

The drive assembly shown generally at 50 includes two drive buttons 52-52 that move reciprocally a selected distance. These drive buttons extend into drive openings 62-62 on the oscillating mounting plate. The oscillating contact element in the embodiment shown has a frequency within the range of 20 Hz to 1 kHz, with a preferred value range of 80-200 Hz. As indicated above, the action of the drive assembly moves the mounting plate 60 parallel with mounting plate 58, so that the oscillating contact element 59 moves parallel to the length of the adjacent fixed contact element 57. In the embodiment shown, the contact element and the mounting plates are made from stainless steel, although the contact elements could also be coated with a compliant material or be composed entirely of compliant material, such as shown at 63 in Figure 14, or the contact elements could be replaced by bristle brush tufts or the like.

In the embodiment shown, a center-to-center distance of approximately 0,32cm (0.125 inches) results in a separation between 0,23 cm (0.09 inches) wide contact elements 57 and 59 of approximately 0,09 cm (0.035 inches). Contact element 59 moves reciprocally over a total distance in a range of 0,05 cm to 0,20 cm (0.02 inches to 0.08 inches), with a preferred value of approximately 0,10 cm (0.040 inches) 0,05 cm (+/- 0.020 inches) from its neutral position to its peak position) along contact element 57. The surface finish of the contact elements 57 and 59 is such that the skin primarily moves in contact with the contact elements. A surface roughness range of 0,13 µm to 0,51 µm (5 to 20 microinches) is effective, with a preferred value of 0,25 µm (10 microinches). The surfaces must be sufficiently rough that the motion of the contact elements is transferred to the skin with minimal or no slippage. If the surface is too smooth, the skin could be abraded. The contact element could be an elastomer or a closed cell foam. It could be a knobby surface or even fingers.

In operation of the embodiment of Figure 6, the edge faces of the contact elements will be positioned lightly against the skin surface and the device activated by,a switch. The contact element 59 begins to oscillate. The device is then moved at a slow rate across the skin surface, for instance two centimeters per second. The device operates with a ratio of peak amplitude to the space (distance) between adjacent skin contacting elements of typically 0.57. With that action, shear forces are applied to the skin, with sufficient amplitude to slightly distort the skin and force open the pores, but low enough to minimize any skin stretching.

At the above-noted frequency range, with a minimum of 20 Hz, each pore opening is deformed approximately 10 times per second. At higher frequencies, the number of deformations per second would be proportionately greater. Alternating shear stress in the tissue surrounding the infundibulum is produced, with the adhesion between the sebaceous plug and the infundibular wall being weakened or significantly reduced, so that the plug is essentially loosened in the pore.

While the aspect of Figure 6 shows one fixed contact element and one movable contact element, it should be recognized that a plurality of fixed contact elements and oscillating contact elements could be used to provide a wider coverage for the device. In the case of a plurality of contact elements, the movable multiple contact element(s) are interdigitated with the fixed contact element(s) and are driven in a ganged manner.

In addition, both contact elements can be driven, preferably in equal and opposite directions of motion with respect to each other. A peak amplitude of 0,05 cm (0.02 inches) for each of two moving elements would result in a peak amplitude of relative motion of 0,1 cm (0.04 inch).

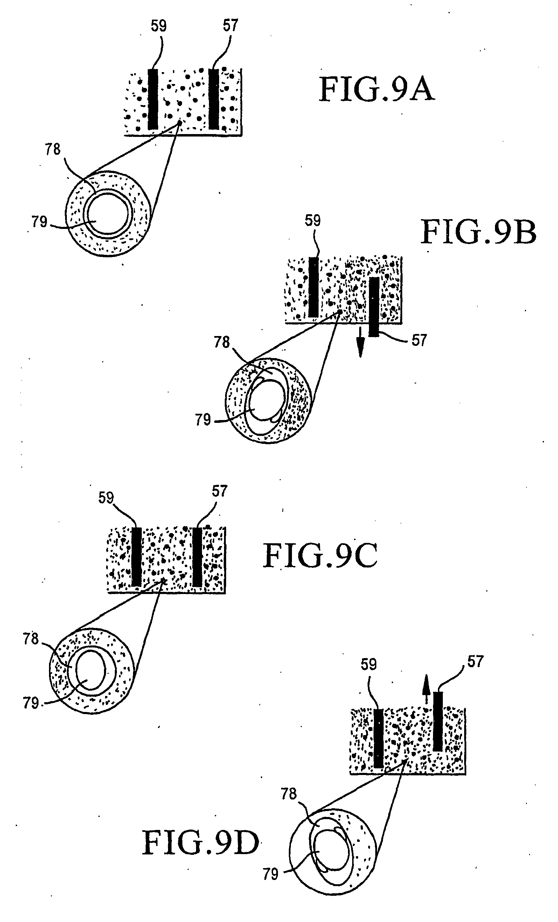

Figures 9A-9D show the action on the skin and a sebaceous plug with the shear aspect of Figures 6-8. Figure 9A shows a pore 78 blocked by a sebaceous plug 79 therein. The contact elements are in a neutral position. The movable contact element will then be moved in one direction, in parallel with the fixed contact element, which distorts the sebaceous plug (Figure 9B). The movable contact element and mounting plate combination is then reversed and returns to the neutral position. This is shown in Figure 9C. The movable contact element continues in the opposite direction, which deforms the sebaceous plug in the opposite direction (Figure 9D). This is accomplished at the specified frequency. While generally this "double" motion is preferred, it is possible to move the movable contact in one direction only relative to the neutral/rest position. Continued repetitive action dislodges or releases the sebaceous plug from the pore walls. The device is slowly moved across the surface of the skin, producing the above results over an entire skin area.

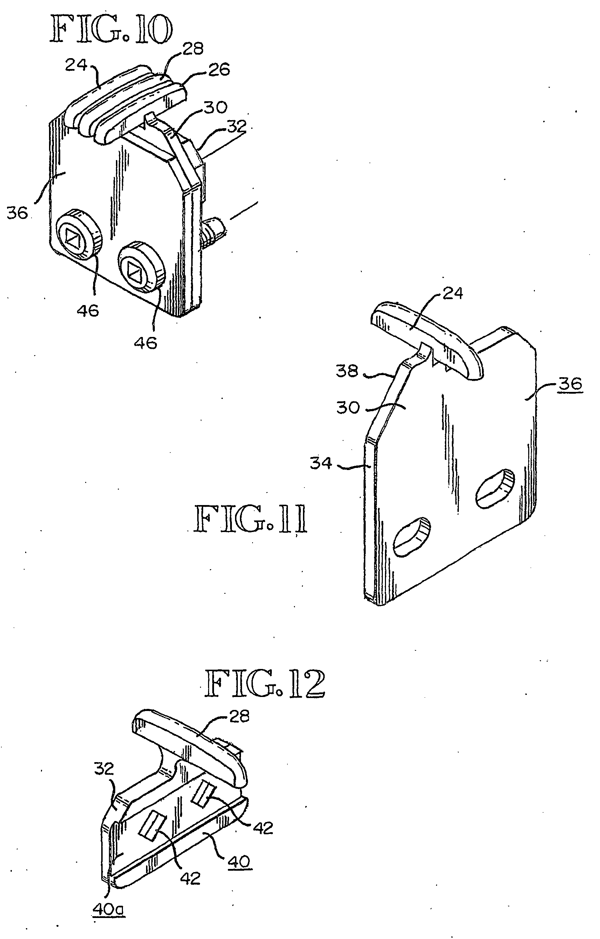

An alternative mechanical arrangement is shown in Figures 10-12. It includes two fixed skin contact elements 24 and 26 and an intermediate oscillating contact element 28. The configuration of the elements in the tension/compression arrangement is similar to that of the elements of the aspect of Figures 6-8, although the "shear" action of the aspect of Figures 6-8 is generally preferred. In both aspects, the differential strain on the skin produced by the mechanical action is sufficient to result in a breaking away of the plug from the skin, due to the difference in elasticity between the plug material and the skin.

In the embodiment of Figures 10-12, the individual contact elements 24, 26 and 28 are each mounted on mounting plates. The fixed contact elements 24, 26 are mounted on mounting plates 30-30 (Figure 11), while the oscillating contact element 28 is mounted on mounting plate 32 (Figure 12). The contact elements are narrow and somewhat elongated and are shown in detail in Figures 11 and 12. In the embodiment shown, the contact elements are typically approximately 30 cm (0.800 inches) long and approximately 0,23 cm (0.090 inches) thick. The edges of each contact element are beveled; giving, each contact element a somewhat rounded end face, while the respective ends are also rounded, as shown in the drawings. The mounting plate 30 for the fixed contact element is approximately 3,00 cm (1.18 inches) wide. The upper corners of mounting plate 30 are both cut off, at a 36° angle. The height of the contact element is somewhat less 3,18 cm ((1.25 inches)) on one side of the contact element relative to the other side 3,56 cm ((1.4 inches)).

In the embodiment shown, the fixed contact elements 24, 26 are mounted cross-wise (perpendicular) to the mounting plate, approximately 1,27 cm (0.5 inches) from one edge 34 thereof. The fixed contact elements are offset laterally, such that they extend approximately 0,80 cm (0.316 inches) from one surface 36 of mounting plate 30, and approximately 1,03 cm (0.406 inches) from the opposing surface 38.

Figure 12 shows the combination of contact element 28 and mounting plate 32 for the oscillating contact element. Mounting plate 32 is, in the embodiment shown, substantially rectangular, with a length of 2,54 cm (1.0 inches) and a height of 1,27 cm (0.5 inches). The upper corners of mounting plate 32 are cut off, i.e. beveled, at an angle of 45°, while the lower corners are rounded.

Oscillating contact element 28 is mounted perpendicularly to mounting plate 32 on the upper edge thereof. The upper edge of contact element 28 is approximately 0,76cm (0.298 inches) above the upper edge of mounting plate 32 and is offset, so that it extends approximately 1,20 cm (0.472 inches) from surface 40 of mounting plate 32. Mounting plate 32 includes two drive holes 42-42 therethrough; so that the mounting plate 32 and contact element 28 can be moved back and forth by a driver mechanism discussed below. In the embodiment shown, the drive holes 42 are approximately square, 0,40 cm (0.154 inches) on each side.

The oscillating contact element 28 on its mounting plate 32 and the fixed contact elements 24 and 26 on their mounting plates 30 are then positioned immediately adjacent to each other, with the two fixed contact element assemblies being back-to-back, but reversed, as shown in Figure 10. The three contact elements 24, 26 and 28 are thus substantially in registry. The three assemblies are held together and attached to the driver by two connecting screws 46-46. The oscillating contact element assembly is movable by means of a driver that moves it back and forth, such that the mounting plate 32 moves parallel to mounting plates 30, and contact element 28 moves alternately toward and away from contact elements 24 and 26.

A drive assembly similar to that shown at 50 in Figure 6 includes two drive buttons which move back and forth a selected distance. These drive buttons connect to the drive holes 42-42 on the oscillating mounting plate 32. The oscillating contact element in the embodiment shown moves at a frequency within the range of 20 Hz to 1 kHz, with a preferred range of 80-200 Hz. As indicated above, the action of the drive assembly moves the mounting plate 32 parallel with mounting plates 30, so that the oscillating contact element 28 moves toward (or away from) one adjacent fixed contact element 24 and away from (toward) the other adjacent fixed contact element 26.

In the embodiment shown, contact elements 24 and 26 are separated by a center-to-center distance of approximately 0,71 cm (0.280 inches) and contact element 28 moves reciprocally over a peak-to-peak distance of approximately 0,38 cm (0.150 inches) between contact elements 24 and 26. Movement between a neutral/rest position and a peak distance (one direction) and back to neutral is also possible. In operation of the embodiment of Figures 10-12, the edge faces of the contact elements will be positioned lightly against the skin surface and the device activated by a switch. The contact element 28 begins to oscillate. The device is then moved at a slow rate across the skin surface, for instance two centimeters per second. With that action, shear forces of tension and compression are applied to the skin, with sufficient amplitude to slightly force open the pores, but low enough to minimize any skin stretching or deformation. In this embodiment, the peak amplitude of motion is approximately 39% of the spacing between adjacent contact elements.

At the above-noted frequency range, with a minimum of 20 Hz, each pore opening is deformed approximately 10 times per second. At higher frequencies, the number of deformations per second would be proportionately greater. Alternating tension and compression stress in the tissue surrounding the infundibulum results, with the adhesion between the sebaceous plug and the infundibular wall being weakened or significantly reduced, so that the plug becomes essentially loose in the pore.

While the embodiment of Figures 10-12 shows two fixed contact elements and one movable contact element, it should be recognized that only one fixed contact element could be used, or a plurality of fixed contact elements and oscillating contact elements can be used to provide a slightly wider coverage. In the case of a plurality of contact elements, the moving multiple contact element(s) are interdigitated with the fixed contact element(s), and are driven in a ganged manner.

In addition, both contact elements can be driven, preferably in equal and opposite directions of motion with respect to each other. A peak amplitude of 0,05 cm (0.02 inches) for each of two moving elements would result in a peak amplitude of relative motion of 0,10 cm (0.04 inches).

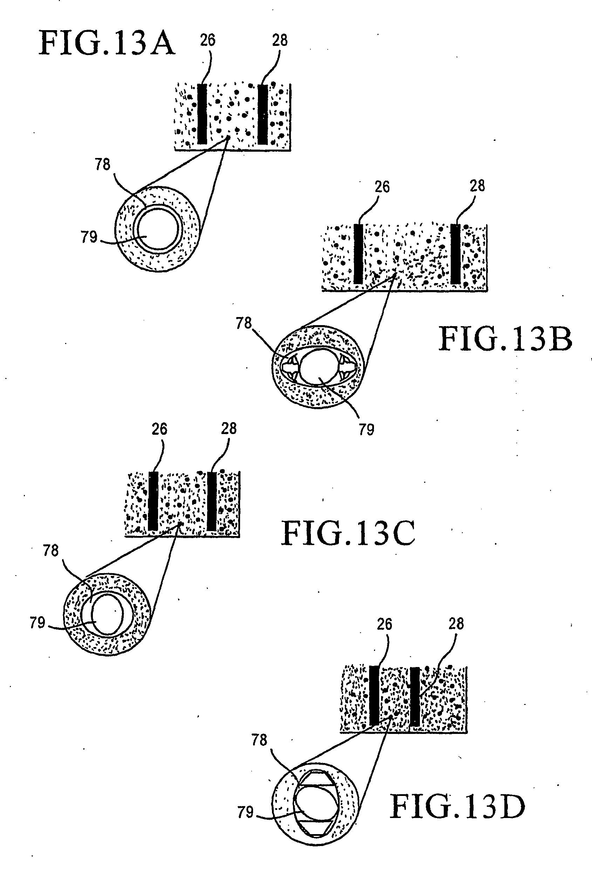

Figures 13A-13D show action on a pore with a sebaceous plug with the tension/compression arrangement of Figures 10-12.

Figure 13A shows a pore 78 blocked by a sebaceous plug 79 therein. The contact elements are in a neutral position. The movable contact element will then be moved in one direction, perpendicularly away from the fixed element, which deforms the sebaceous plug and causes deformation of the pore in one direction (Figure 13B). The motion is then reversed and returns to the neutral position, relaxing the force between the sebaceous plug and the acroinfundibulum, as shown in Figure 13C. The movable contact element will then be moved in the opposite direction, perpendicularly away from the fixed element, which also deforms the sebaceous plug opposite direction (Figure 13D). This sequence is accomplished at a frequency within the range of 20-1,000 Hz, and preferably in the range of 80-200 Hz. Continued action dislodges or releases the sebaceous plug from the pore walls. The user slowly moves the device across the surface of the skin, producing the above results over an entire skin area.

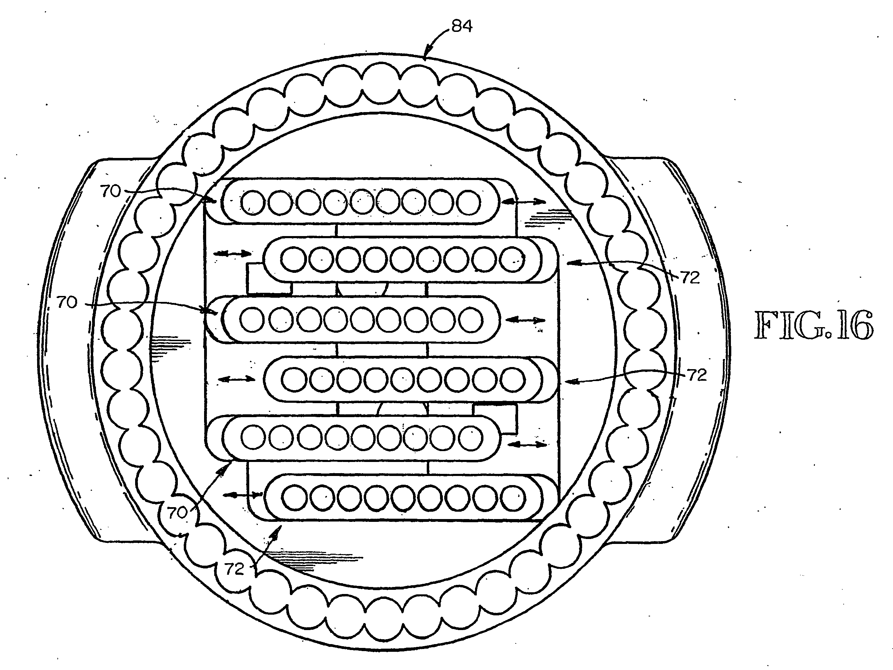

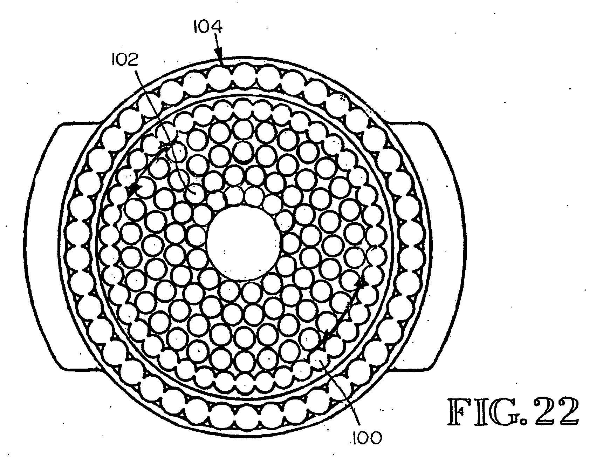

A further alternate mechanical configuration is shown in Figures 15-22. These configurations operate on substantially the same principles as the devices described above, but have contact elements composed of bristle tufts. In these embodiments, the base portions holding the bristle tufts are analogous to the mounting plates described above. Instead of rigid or compliant solid contact elements, a plurality of bristle tufts are employed. Each tuft is further composed of a plurality of filaments or bristles. The bristles may be made from any material suitable for the application, with the preferred material being Nylon 612. The diameter of each bristle is in the range of 0,05 to 0,13 mm (2 to 5 mils) with a preferred diameter of 0,76 mm (3 mils), and of lengths in the range 0,64 to 1,52 cm (0.25 to 0.60 inches), with a preferred length of 1,10 cm (0.43 inches) in length.

The base of the tuft has a diameter in the range of 1,01 mm to 2,54 mm (40 to 100 mils) with a preferred diameter of 1,52 mm (60 mils) for the tufts of the fixed and moving interior bristle tuft rows and a preferred diameter of 2,03 mm (80 mils) for the fixed exterior bristle tuft row. The diameter and length of the bristles determine their stiffness. Using the same material, larger diameter bristles are stiffer than smaller diameter bristles. Generally longer bristles are softer than shorter bristles. The material used to make the bristles also dictates the stiffness character of the bristles. Additionally, the rows can be made with individual tufts having a different number of bristles. Generally, having more bristles of a smaller diameter in a tuft will produce a softer sensation.

Tufts of 0,01 cm (0.003 inches) diameter Nylon 612 bristles 0,110 cm (0.43 inches) in length produce a lateral stiffness which works well in moving the skin within Region I and the lower part of Region II of Figure 4. Such tufts produce a lateral spring constant of approximately 3,9 g/cm (10 grams/inch) at a displacement of 0,15 cm (0.06); i.e. a lateral displacement of 0,15 cm (0.06) of the end of a tuft results when a lateral force of 0.6 grams is applied to the end of the tuft relative to the base.

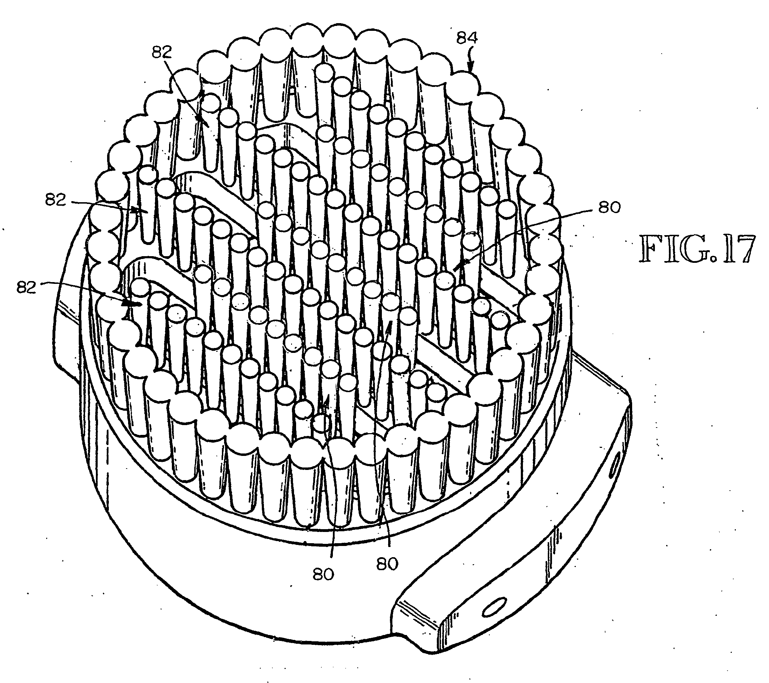

Figures 15-18 show an embodiment using linear motion of the bristle rows. In Figures 15-16, both sets of bristle rows (first set of three rows 70-70, second set of three rows 72-72) move with respect to each other, while in Figures 17-18, one set of four rows 82-82 is fixed and the other set of three rows 80-80 moves. In both embodiments, the rows of moving/fixed bristle tufts are surrounded by a circle of bristle tufts 84, the circle of bristles 84 being fixed and functioning like a curtain to keep cleanser/water on the skin.

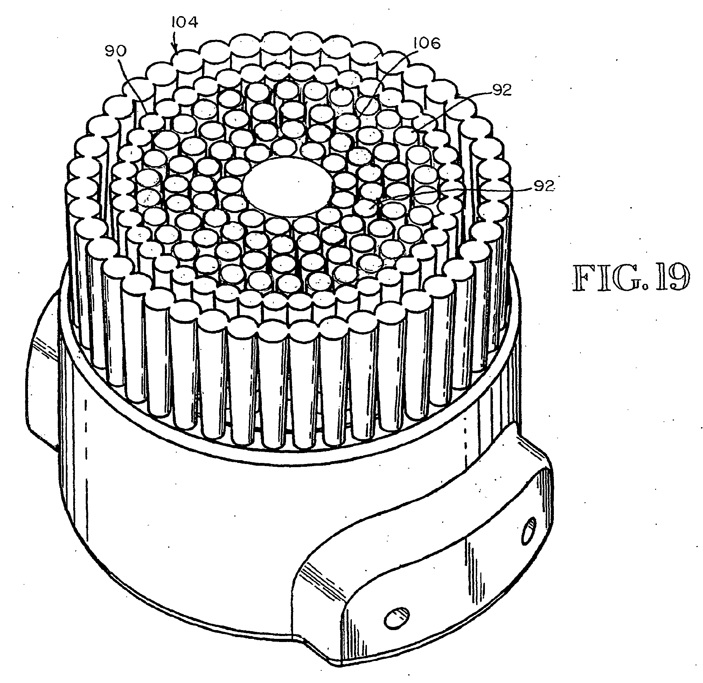

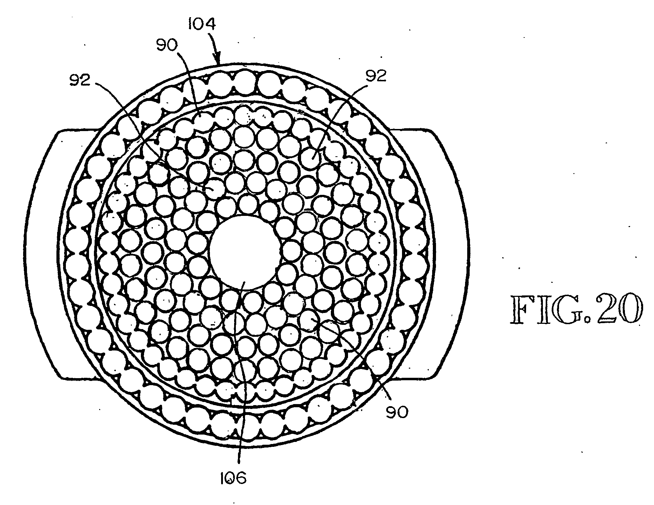

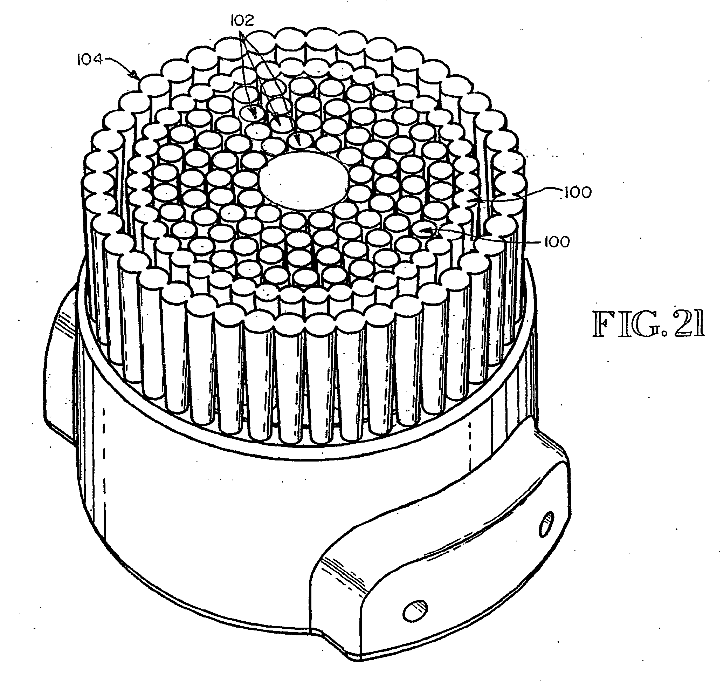

In another embodiment shown in Figures 19-22, the row(s) of bristle tufts are circular and move in an arcuate manner with the axis of rotation perpendicular to the surface of the skin. Figures 19-20 show an embodiment in which both sets of circular bristle rows (two rows 90-90 and two rows 92-92) move with respect to each other, while Figures 21-22 show an embodiment in which one set of two rows 100-100 moves and the other set of three rows 102-102 is fixed. In each case, one row each of 90 and 92 bristle rows and one row each of 100 and 102 bristle rows would likely be sufficient for cleansing action. Additional rows beyond that shown could also work. In both of these embodiments, the rows of bristles are encircled by a circle of fixed bristles 104, acting as a curtain for liquid, etc. In the embodiment of Figures 19 and 20, there is also a circle of fixed bristles 106 inside of the circle of rows 90 and 92.

The adjacent rows of bristle tufts for the devices shown in Figures 15-22 move relative to each other at an amplitude sufficient to deform the skin in region I and slightly into region II of Figure 4 as shown to produce the cleansing action.

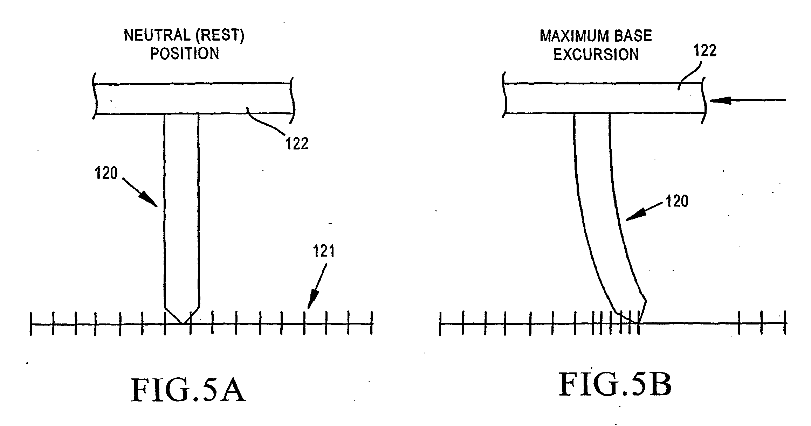

Figure 5A shows the cleansing action of bristles with a single tuft of bristles 120 against the skin 121 when base 122 is at rest (neutral), while Figure 5B shows the tuft 120 when the base 122 is at a maximum excursion. The skin 121 is indicated with "tick" marks on a horizontal line, with the tick spacing showing relative deformation of the skin.

In Figure 5A, the uniform spacing of the tick marks indicates that there is no deformation of the skin when the bristles are in the rest position. Figure 5B shows that the skin has been compressed slightly by the bristles in the direction of motion of the base, and stretched slightly behind the tips of the bristles. In typical operation, both the bristles and the skin deform, and there is relatively little slippage of the bristles on the skin. As the tuft base moves from its rest (neutral) position, the skin deformation increases, as does its modulus, until the restorative force of the skin just balances that of the bristles.

Typical peak-to-peak amplitudes measured at the base of the bristle tufts of 0,13 cm to 0,64 cm (0.05 inches to 0.25 inches) can be used with rows having center-to-center spacing of 0,25 cm to 0,64 cm (0.10-0.25 inches). This results in the peak amplitude (50% of peak-to-peak amplitude) of typically 40%, and in a range of 10% to 100% of the center-to-center spacing between adjacent rows of tufts. At high amplitudes, the bristles may also slide across the surface, especially if the brush is used with lubricating elements.

Referring now again to Figures 19-22, lubricating fluid can be supplied by the device, for example, through a central port 106 shown in Figures 19 and 20. Centripetal force tends to spread the emitted fluid onto the bristles, supplying relatively uniform wetting. The fluid is contained by the bristle curtain 104.

In the case of rotational configurations such as shown in Figures 19-22, the linear amplitude of motion is larger for the outer rings. The center-to-center spacing can be adjusted among the rows to maintain an approximately constant ratio of amplitude to inter-element spacing.

The bristle rows described above can also be replaced with flexible members, such as an elastomer or closed cell foam.

It is also possible to combine the advantages of the differential shear mode and tension/compression modes described above into a compound motion, for example, elliptical.

It is also possible to apply bi-directional motion to the skin via a single set of contact elements for cleaning or clearing the infundibular opening. Unlike the cases above in which there is a differential reciprocating motion between adjacent contact elements, the use of a single set of elements relies on inertia of the skin to effect a differential force on the pore openings. The single set of moving contact elements, such as a row of bristles, forces the skin immediately adjacent to it to move. This movement is coupled to skin regions somewhat distant through the skin's elasticity. However, skin also has inertia which resists motion, thereby producing a shear force in the direction of movement. This shear force decreases at greater distances from the moving contact elements.

Applying bi-directional reciprocating movement via a single set of contact elements is generally not as effective as using adjacent contact elements arranged to apply tension/compression or shear between them.

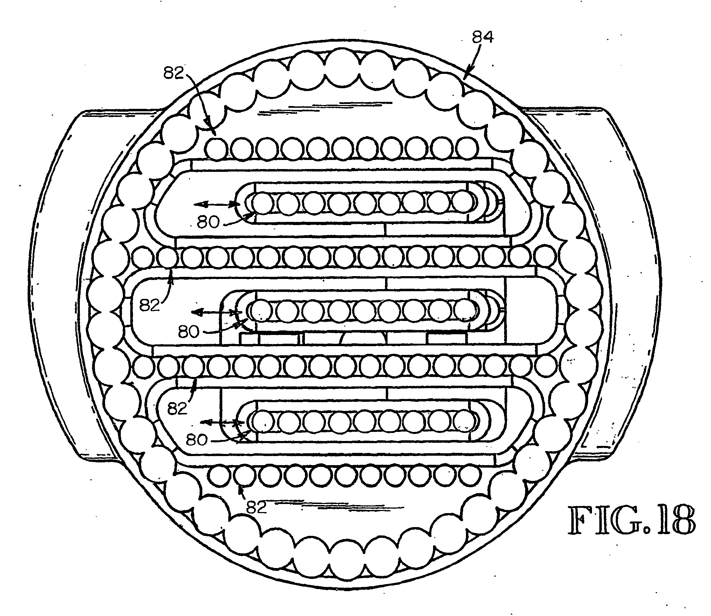

The single set of reciprocating contact elements can be implemented in a linear manner, such as the device of Figures 17 and 18, with all linear bristles moving in unison. This single set reciprocating motion can also be implemented in an arcuate manner, such as the device of Figures 21-22, with all rows of bristles moving in unison. Where the contacting element comprises rigid or compliant material, the peak-to-peak amplitude of movement will be within the range of 0,1cm to 0,40 cm (0.04-0.150 inches), preferably 0,23 cm (0.09 inches); if the contacting element is a row of bristles, the peak-to-peak amplitude of movement is within the range of 0,05 cm to 0,41 cm (0.020 to 0.160 inches), preferably 0.082 inches.

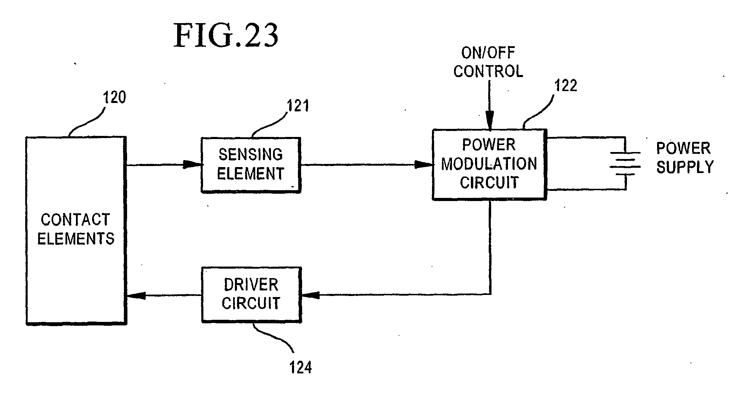

Figure 23 shows a schematic block diagram for a control means for controlling the amplitude of the moving portions of the contact elements 120. The control means is composed of a sensing element 121, a power modulation circuit 122, and a driver circuit 124. The sensing element 121 senses the amount of pressure applied to the skin by the contact elements 120 and applies a signal to the power modulation circuit 122. The power modulation circuit 122 uses said signal to modulate the power of the driver circuit 124 and amplitude of the contact elements 120.

The control means operates in a plurality of operating modes with a preferred number of three operating modes. Proper operation of the apparatus requires that the pressure applied to the skin by the applicator remains in given range.

When pressure applied by the contact, elements to the skin is below the lower threshold for proper operation of the apparatus, the amplitude of the applicator with the contact elements is substantially reduced from its nominal amplitude. This reduces the likelihood of splashing of fluids or cleaning agents when the applicator is not in contact with the skin.

When pressure applied by the applicator to the skin is above the lower threshold for proper operation of the apparatus, but below the upper threshold, the applicator is driven at nominal amplitude.

When pressure applied to the skin by the applicator is above the upper threshold for proper operation the amplitude of the applicator with the contact elements is substantially reduced from its normal amplitude or, preferably, stopped altogether. An alternative is to interrupt the power to the contact elements at a low frequency, for example 2-10Hz, in order to create an audible or tactile feedback to the user to reduce the pressure. This excess pressure feedback signal reduces the likelihood that the applicator will cause too much motion of the skin.

The above-described control means provides not only safety and convenience, but also provides feedback to the user to maintain applicator pressure in the range for proper operation of the apparatus.

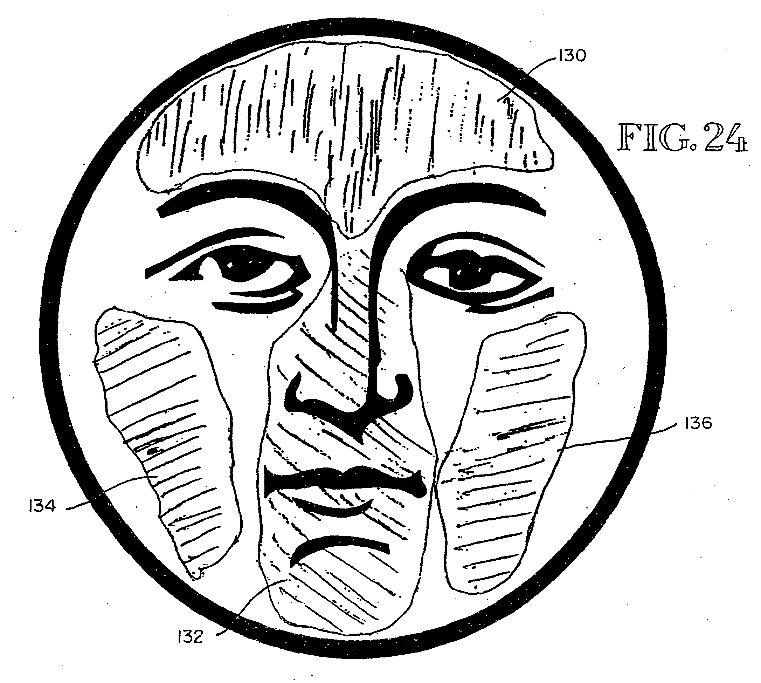

Figure 24 shows four regions of the face that differ in topology and degree of sebaceous secretion. Generally the face is divided into two distinct regions, the so-called "T-zone" 130 and 132, and the outer cheek areas 134, 136. The T-zone is the part of the face consisting of the forehead, nose and the area around the mouth, including the chin. It is so named because it is shaped like the letter T.

Often the T-zone is more prone to acne, as the percentage of sebum glands in this area tends to be higher than on the outer cheeks. An important component of the present invention, therefore, is a timer means to assist the user in properly treating the differing zones of the face, according to the typical incidence of acne in that area, without over- or under-treating the area. The total treatment time may be from 30 seconds to two minutes and preferably one minute. Further, the total time may be subdivided into two or more and preferably four time periods. In the present invention, the first time period is 20 seconds for treatment of the forehead 130; the second time period is 20 seconds for treatment of the nose, perioral area and chin 132; and 10 seconds for each outer cheek areas 134, 136.

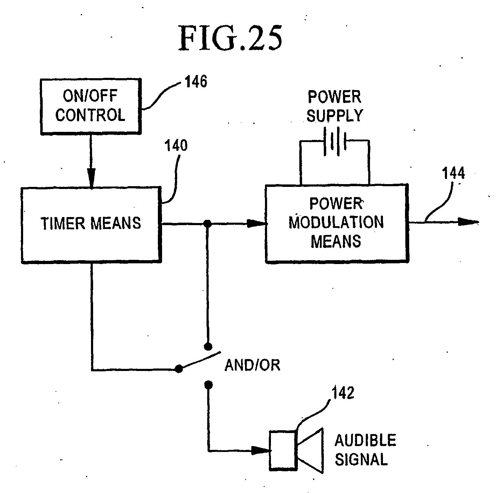

A timer means 140 (Figure 25) prompts the user by providing either an audible signal 142 or a detectable change in the motion of the moving contacting element, or both simultaneously. This prompt instructs the user when the preferred treatment time has elapsed for each area of the face. The timer can be enabled or disabled after the apparatus is turned on, by means of an on/off switch 146 being pressed on for a selected period of time. A first audible signal can be used to indicate that the timer has been enabled and a second audible signal to indicate that the timer has been disabled.

In summary, applying differential motion locally to the infundibular (pore) opening results in the clearing of sebaceous plugs from the acroinfundibulum (top of the pore). The differential motion, whether linear, arcuate or elliptical, applies forces to the interface between the comedone (sebaceous plug) and the surrounding tissue, thus breaking the adhesion between the acroinfundibular wall and the sebaceous plug.

A bi-directional, return-to-center motion generally provides better cleaning than unidirectional motion due to the nature of the sebaceous plug, i.e. the sebaceous plug can be thought of as a generally disorganized matrix of flat, brick-like corneocytes embedded in a "mortar" of oxidized sebum lipids. Adhesion of the plug to the wall of the acroinfundibulum is thought to be caused by a combination of oxidized sebum and ceramide lipids. Because the orientation of the corneocytes is not completely random relative to the wall of the acroinfundibulum, it is possible that unidirectional motion alone would eliminate some of the adhesion but may be insufficient to loosen the sebum plug. The preferred embodiment of the invention applies bi-directional motion such that most or all of the corneocytes are subject to adhesion-breaking stresses irrespective of their orientation.

Limiting the amplitude of bi-directional motion to an extent which generally maintains the skin in a region of low strain is also beneficial. High amplitude bi-directional or uni-directional motion places the collagen fibers in a higher strain condition.

In use, our invention applies cyclic deformation and relaxation many times per second to the skin surrounding the acroinfundibulum and any sebaceous plug. The repetition of differential vibratory cycles supplies a therapeutic effect by gradually breaking the adhesion between the acroinfundibulum and the sebaceous plug.

The present invention is intended to operate in a frequency range of 20-1,000 Hz. A preferred range is 80-200 Hz. Below 80 Hz, the vibration rate is less than optimal and the mechanical implementation is more difficult. Above 200 Hz, a strong tickle reaction, usually unpleasant, occurs in the nose region. Assuming a 1 cm width of active surface of the device operating at the minimum frequency, moving the device across the skin surface linearly at 2 cm/sec would result in each pore experiencing 10 deformation cycles, many more times than would be practicable by any manual technique. At higher frequencies the number of deformations per stroke of the appliance would be proportionally greater.

There are two basic modes of differential movement that can be applied: shear and tension/compression. The shear mode device applies a linear differential motion via narrow elements which contact the skin, and which move in the direction of their length with respect to each other. The device typically applies a sinusoidal oscillation to adjacent contact elements. The arrangement includes two contact element assemblies. The device moves the contact elements in parallel to each other along their long axis. Sufficient frictional forces between the surface of the contact elements and the skin surface will transfer this motion to the skin, creating a shear action on the skin between them as shown in Figures 9A-9D.

The tension/compression mode device, in contrast to shear mode, moves the contact elements toward and away from each other. The oscillations are perpendicular to the long axis of the contact elements (i.e. one element moving toward one neighbor and away from its other neighbor), thus creating alternating tension and compression stress in the tissue surrounding the infundibulum. Sufficient frictional forces between the surface of the contact elements and the skin surface will transfer this motion to the skin as shown in Figures 13A-13D.

Alternatively to one contact element moving, both contact elements may move with respect to the device body, and counter to one another.

The skin contracting elements can be rigid or flexible. Rigid surfaces can be made from stainless steel and plastic. Flexible contacting surfaces can include bristles, elastomers and soft compliant foam. The surfaces should have sufficient roughness in order to transfer the motion to the skin without slippage, or minimizing such slippage. Additionally, the proper degree of surface roughness assures good lamellar action (transfer of lubricant from wet to dry portion of skin by interstitial spaces in the contacting surface). If the surface finish is too smooth the lubrication is wiped off and the contacting surface runs dry against skin and may cause unwanted abrasion of the skin. This surface roughness can be in the range of 5 to 20 micro-inches and preferably is 10 micro-inches.

Multiple contacting elements can be included, such that a set of skin contacting elements moving in one direction are interdigitated between a set of stationary skin contacting elements, or skin contacting elements moving in the opposing direction. Figure 15 shows a device with a double pair of skin contacting elements, and is a derivative of the device with a single pair of skin contacting elements shown in Figure 6. In the case shown in Figure 15, each of two sets of skin contacting elements consists of three rows of bristle tufts. Each set of bristle tuft rows moves in relative opposition, surrounded by a fixed circular row of fixed bristle tufts, which serve to minimize splashing and to control the contact of the moving bristle tips onto the skin. The bristle tufts are designed so that the interdigitated row movement results in sufficient force on the skin to maintain acroinfundibular openings. This action is similar to fingers of left and right hands interlocking while hand washing.

The model shown has three rows of bristles in each of the two interdigitated sets, but the number of bristle rows could vary from a single row to as many as practical for the desired surface area.

Additionally, the motion of the bristle tuft row(s) can be linear, arcuate or elliptical along the plane of the skin with the axis of rotation perpendicular to the skin.

The magnitude of reciprocal force applied to the skin is primarily determined by the stiffness of bristle tufts to lateral deformation, the length and width of the bristle rows, spacing between bristle rows, amplitude of interdigitated motion, and the pressure applied by the user.

The effects on the skin by the movement of the contacting elements can also be modified with the use of a skin lubricant. The lubricant can be water, soapy water, another skin cleaning agent, a lotion or gel. More lubrication results in more sliding action of the bristle tips across the skin, and less deforming action applied to the skin. The sliding action across the skin serves to remove skin surface debris. The debris includes sebum, triglycerides and fatty acids, desquamatized corneocytes and accumulated dirt and environmental materials.

Thus, the present invention provides either mechanical energy in a shear mode or tension/compression mode or a combination (elliptical) in order to loosen the adhesion between the sebaceous plug and the walls of the pore. Said motion can be produced by contact elements moving either reciprocally linearly, reciprocally arcuately or a combination thereof. The loosened sebaceous plug and any previously blocked lipids from the pores can then be readily removed by rinsing the cleansed area. Such an arrangement results in an effective treatment of early stage acne that prevents the development of more serious acne conditions. In addition, however, the arrangement can be used for effective cleansing of skin when acne is not present. The combination of gentleness and cleansing action produces a desirable, effective cleansing effect on the skin and a "sense" or feel by the user of clean, healthy skin.

## Claims

1. An apparatus for treatment of acne, comprising:
at least two contacting elements (57, 59) having end faces, wherein at least one contacting element is a moving contacting element (59);
a mounting assembly (58, 60) for holding the contacting elements substantially adjacent to each other; and
an assembly (50) for reciprocally moving said at least one moving contacting element;
**characterised in that**, all of the end faces are in the same plane and the end faces of the moving contacting element (59) move in a single plane; and said assembly reciprocally moves said at least one moving contacting element bidirectionally through a neutral position relative to at least one adjacent contacting element, to produce alternating tension and compression of the skin, wherein when the apparatus is positioned so that the end faces of the contacting elements contact the skin, an action on the skin in the plane of a skin area to be treated for acne is produced to remove sebum plugs from skin pores, permitting ready removal thereof from the skin.

2. An apparatus of claim 1, wherein the frequency of movement of the moving contacting element is within a range of 20 Hz to 1 KHz.

3. An apparatus of claim 2, wherein the frequency is preferably within the range of 80-200 Hz.

4. An apparatus of claim 1, wherein the contacting elements comprise a rigid material.

5. An apparatus of claim 4, in which the contacting elements have a contacting surface with a roughness within a range of 0.127 µm to 0.508µm (5 to 20 microinches).

6. An apparatus of claim 4, in which the roughness is approximately 0.254µm (10 microinches).

7. An apparatus of claim 4, wherein the ratio of peak amplitude of movement of the moving contacting element is within a range of 15%-120% of the distance between adjacent contacting elements moving with respect to each other.

8. An apparatus of claim 7, wherein said ratio is approximately 60%.

9. An apparatus of claim 1, wherein the contacting elements comprise, respectively, rows (70, 72, 80, 82, 90, 92, 100, 102) of bristle tufts.

10. An apparatus of claim 9, wherein the bristles have a diameter in the range of 0.0508 to 0.127mm (2 to 5 mils) and a length in the range of 6.35 to 15.24mm (0.250 to 0.600 inches), and wherein the bristle material has a flexural modulus in the range of 689MPa to 6.89KPa (100 to 1000 kpsi).

11. An apparatus of claim 9, wherein the tufts have mechanical characteristics such that a displacement of 1.58 mm (0.062 inches) at the base of a tuft causes a force in the range of 0.3 grams to 1.4 grams, to be exerted on the skin at the end of said tuft.

12. An apparatus of claim 11, wherein the force is approximately 0.61 grams.

13. An apparatus of claim 9, wherein the peak amplitude of motion at the base of one bristle tuft with respect to that of an adjacent contacting element is in the range of 10%-125% of the center-to-center distance between adjacent contacting elements moving with respect to each other.

14. An apparatus of claim 13, wherein said peak amplitude of motion at the base of one bristle tuft (70) with respect to that of an adjacent element (72) is approximately 45%.

15. An apparatus of claim 1, wherein the contacting elements comprise a compliant material.

16. An apparatus of claim 15, wherein the contacting elements comprise an elastomeric solid material.

17. An apparatus of claim 15, wherein the contacting elements comprise a foam material.

18. An apparatus of claim 1, wherein the movement of the moving contacting element reciprocally places the skin in alternating tension and compression.

19. An apparatus of claim 18, wherein the peak amplitude of movement of any moving contacting element is a linear displacement with respect to a point on an adjacent contacting element, and where said linear displacement is in the range of 10% to 60% of the distance between adjacent contacting elements when in their neutral position.

20. An apparatus of claim 19, wherein said linear displacement is approximately 40%.

21. An apparatus of claim 18, wherein the contacting elements are composed of a rigid material.

22. An apparatus of claim 21, wherein the contacting elements have a surface finish in the range of 0.127 µm to 0.508 µm (5 to 20 microinches).

23. An apparatus of claim 22, wherein the surface finish is approximately 0.254 µm (10 microinches).

24. An apparatus of claim 1, wherein the motion of the moving contacting elements is controlled by a control means (121, 122, 124) whereby the amplitude of motion of the moving contacting elements is substantially reduced when the contacting elements are not in contact with the skin and whereby the amplitude of motion of the moveable contacting elements is at the preferred magnitude when the contacting elements are applied to the skin with correct pressure for optimal operation and whereby the amplitude of motion of the moveable contacting elements is modified in a manner detectible by the user when the contacting elements are applied to the skin with greater than the correct pressure.

25. An apparatus of claim 1, including a fluid supplying structure (106) positioned adjacent to the contacting elements and wherein said structure supplies a fluid which contacts the skin during operation.

26. An apparatus of claim 1, including a fluid- containing structure (106) positioned adjacent the contacting elements and wherein said fluid-containing structure substantially prevents fluid from being expelled from the apparatus by movement of the contacting elements.

27. An apparatus of claim 26, wherein said fluid- containing structure consists of bristle tufts having a free end in approximately the same plane as that of the contacting elements.

28. An apparatus of claim 1, including a control assembly (121, 122, 124) for controlling pressure applied to the skin so as to minimize the likelihood that excess skin pressure is exerted on the moving contacting elements.

29. An apparatus of claim 1, wherein said contacting elements are removable and replaceable.

30. An apparatus of claim 1, having a timing means (140), responsive to the device being turned on, for producing successive indications of intervals of elapsed time, wherein the intervals of elapsed time are related to desired times for treating portions of the face, wherein the successive indications of time are provided within a pre-established set period of time during which the apparatus is operating; and means for maintaining the status of the timing means for a selected period of time after the apparatus has been turned off during said pre-established period of time after the apparatus has been turned on.

31. An apparatus of claim 30, wherein the successive indications of time are provided within a pre-established set period of time during which the apparatus is operating.

32. An apparatus of claim 31, wherein the set period of time is approximately one minute and wherein the selected intervals of time are approximately 10 to 20 seconds.

33. An apparatus of claim 30, wherein the indications are a selected one of a) an audible signal and b) a detectable change in moving contacting element movement.

34. An apparatus of claim 30, wherein the indications comprise both an audible signal and a detectable change in moving contacting element movement.

35. An apparatus of claim 30, including means (146) for changing the state of the timing means between enabled and disabled after the apparatus is turned on.

36. An apparatus of claim 35, wherein the changing means is responsive to an on/off switch being pressed on for a selected period of time.

37. An apparatus of claim 36, wherein a first audible signal indicates that the timing means has been enabled and a second audible signal different from the first audible signal indicates that the timing means has been disabled.

38. An apparatus of claim 9, wherein the rows of bristle tufts comprise a plurality of straight rows, alternating ones of which (a) move (80) and (b) remain fixed (82) in position.

39. An apparatus of claim 38, including a further row of fixed bristle tufts (84) surrounding the plurality of straight rows.

40. An apparatus of claim 9, wherein the tows of bristle tufts comprise a plurality of straight rows (70, 72), alternating ones which move in opposing directions.

41. An apparatus of claim 40, including a further row of fixed bristle tufts (84) surrounding the plurality of straight rows.

42. An apparatus of claim 9, wherein the rows of bristle tufts comprise a plurality of circular rows, alternating ones of which (a) move (90) and (b) remain fixed (92) in position.

43. An apparatus of claim 42, including a further row of fixed bristle tufts (104) surrounding the plurality of circular rows.

44. An apparatus of claim 9, wherein the rows of bristle tufts comprise a plurality of circular rows (100, 102), alternating ones of which move in opposing directions.

45. An apparatus of claim 44, including a further row of bristle tufts (104) surrounding the plurality of circular rows.

## Patentansprüche

1. Vorrichtung zur Behandlung von Akne, die Folgendes aufweist:
mindestens zwei Kontaktierungselemente (57, 59) mit Stirnflächen, wobei mindestens ein Kontaktierungselement ein sich bewegendes Kontaktierungselement (59) ist;
eine Befestigungsanordnung (58, 60) zum Halten der Kontaktierungselemente im Wesentlichen benachbart zueinander; und
eine Anordnung (50) zum Hin- und Herbewegen von dem mindestens einen sich bewegenden Kontaktierungselement;
**dadurch gekennzeichnet, dass** alle Stirnflächen in der gleichen Ebene sind und die Stirnflächen von dem sich bewegenden Kontaktierungselement (59) sich in einer einzigen Ebene bewegen und die Anordnung das mindestens eine sich bewegende Kontaktierungselement in beiden Richtungen durch eine neutrale Position relativ zu dem mindestens einen benachbarten Kontaktierungselement hin und her bewegt, um abwechselnd eine Spannung und eine Kompression der Haut zu erzeugen, wobei, wenn die Vorrichtung derart positioniert ist, dass die Stirnflächen der Kontaktierungselemente mit der Haut in Kontakt sind, eine Wirkung auf die Haut in der Ebene eines Hautbereichs, der wegen Akne behandelt werden soll, erzeugt wird, um Talgpfropfen aus den Hautporen zu entfernen, was deren bequeme Entfernung von der Haut ermöglicht.

2. Vorrichtung gemäß Anspruch 1, wobei die Bewegungsfrequenz des sich bewegenden Kontaktierungselements innerhalb eines Bereiches von 20 Hz bis 1 KHz liegt.

3. Vorrichtung gemäß Anspruch 2, wobei die Frequenz vorzugsweise in einem Bereich von 80 - 200 Hz ist.

4. Vorrichtung gemäß Anspruch 1, wobei die Kontaktierungselemente ein steifes Material aufweisen.

5. Vorrichtung gemäß Anspruch 4, in der die Kontaktierungselemente eine Kontaktfläche mit einer Rauigkeit in einem Bereich von 0,127 µm bis 0,508 µm (5 bis 20 Mikroinch) aufweisen.

6. Vorrichtung gemäß Anspruch 4, in der die Rauigkeit annähernd 0,254 µm (10 Mikroinch) beträgt.

7. Vorrichtung gemäß Anspruch 4, wobei das Verhältnis der Spitzenamplitude der Bewegung des sich bewegenden Kontaktierungselements in einem Bereich von 15 % - 120 % des Abstandes zwischen benachbarten Kontaktierungselementen liegt, die sich zueinander bewegen.

8. Vorrichtung gemäß Anspruch 7, wobei das Verhältnis annähernd 60 % beträgt.

9. Vorrichtung gemäß Anspruch 1, wobei die Kontaktierungselemente jeweils Reihen (70, 72, 80, 82, 90, 92, 100, 102) Borstenbündel aufweisen.

10. Vorrichtung gemäß Anspruch 9, wobei die Borsten einen Durchmesser im Bereich von 0,0508 bis 0,127 mm (2 bis 5 mils) und eine Länge im Bereich von 6,35 bis 15,24 mm (0,250 bis 0,600 Inch) aufweisen und wobei das Borstenmaterial ein Biegemodul im Bereich von 689 MPa bis 6,89 KPa (100 bis 1000 kpsi) aufweist.

11. Vorrichtung gemäß Anspruch 9, wobei die Bündel mechanische Eigenschaften derart aufweisen, dass eine Verschiebung um 1,58 mm (0,062 Inch) an der Basis eines Bündels eine Kraft im Bereich von 0,3 Gramm bis 1,4 Gramm verursacht, die an dem Ende des Bündels auf die Haut ausgeübt wird.

12. Vorrichtung gemäß Anspruch 11, wobei die Kraft annähernd 0,61 Gramm beträgt.

13. Vorrichtung gemäß Anspruch 9, wobei die Spitzenamplitude der Bewegung an der Basis von einem Bürstenbündel in Bezug auf die von einem benachbarten Kontaktierungselement im Bereich von 10 % - 125 % des Mittenabstands zwischen benachbarten Kontaktierungselementen, die sich zueinander bewegen, liegt.

14. Vorrichtung gemäß Anspruch 13, wobei die Spitzenamplitude der Bewegung an der Basis von einem Bürstenbündel (70) in Bezug auf die von einem benachbarten Element (72) annähernd 45 % beträgt.

15. Vorrichtung gemäß Anspruch 1, wobei die Kontaktierungselemente ein nachgiebiges Material aufweisen.

16. Vorrichtung gemäß Anspruch 15, wobei die Kontaktierungselemente ein elastomeres Vollmaterial aufweisen.

17. Vorrichtung gemäß Anspruch 15, wobei die Kontaktierungselemente ein Schaummaterial aufweisen.

18. Vorrichtung gemäß Anspruch 1, wobei die Bewegung des sich bewegenden Kontaktierungselements die Haut wechselseitig unter sich abwechselnde Spannung und Kompression setzt.

19. Vorrichtung gemäß Anspruch 18, wobei die Spitzenamplitude der Bewegung irgendeines der sich bewegenden Kontaktierungselements eine lineare Verschiebung in Bezug auf einen Punkt an einem benachbarten Kontaktierungselement ist und wobei die lineare Verschiebung im Bereich von 10 % bis 60 % des Abstandes zwischen benachbarten Kontaktierungselementen liegt, wenn diese in ihrer neutralen Position sind.

20. Vorrichtung gemäß Anspruch 19, wobei die lineare Verschiebung annähernd 40 % beträgt.

21. Vorrichtung gemäß Anspruch 18, wobei die Kontaktierungselemente aus einem steifen Material bestehen.

22. Vorrichtung gemäß Anspruch 21, wobei die Kontaktierungselemente eine Oberflächenbeschichtung im Bereich von 0,127 µm bis 0,508 µm (5 bis 20 Mikroinch) aufweisen.

23. Vorrichtung gemäß Anspruch 22, wobei die Oberflächenbeschichtung annähernd 0,254 µm (10 Mikroinch) ist.

24. Vorrichtung gemäß Anspruch 1, wobei die Bewegung der sich bewegenden Kontaktierungselements durch eine Steuerungseinrichtung (121, 122, 124) gesteuert wird, wodurch die Amplitude der Bewegung von den sich bewegenden Kontaktierungselementen im Wesentlichen verringert ist, wenn die Kontaktierungselemente nicht in Kontakt mit der Haut sind und wodurch die Amplitude der Bewegung von den beweglichen Kontaktierungselementen in der bevorzugten Größenordnung ist, wenn die Kontaktierungselemente mit dem korrekten Druck für einen optimalen Einsatz auf die Haut angewendet werden und wodurch die Amplitude der Bewegung der beweglichen Kontaktierungselemente auf eine Weise modifiziert wird, die durch den Anwender feststellbar ist, wenn die Kontaktierungselemente mit einem größeren als dem korrekten Druck auf die Haut angelegt werden.

25. Vorrichtung gemäß Anspruch 1, die eine Fluidversorgungsstruktur (106) umfasst, welche benachbart zu den Kontaktierungselementen positioniert ist und wobei die Struktur ein Fluid liefert, welches während des Einsatzes mit der Haut in Kontakt ist.

26. Vorrichtung gemäß Anspruch 1, die eine Fluid enthaltende Struktur (106) umfasst, welche benachbart zu den Kontaktierungselementen positioniert ist und wobei die Fluid enthaltende Struktur im Wesentlichen verhindert, dass Fluid durch die Bewegung der Kontaktierungselemente aus der Vorrichtung ausgestoßen wird.

27. Vorrichtung gemäß Anspruch 26, wobei die Fluid enthaltende Struktur aus Borstenbündeln mit einem freien Ende in annähernd derselben Ebene wie die der Kontaktierungselemente besteht.

28. Vorrichtung gemäß Anspruch 1, die eine Steueranordnung (121, 122, 124) zur Steuerung des auf die Haut ausgeübten Drucks aufweist, um so die Wahrscheinlichkeit gering zu halten, dass übermäßiger Hautdruck auf die sich bewegenden Kontaktierungselemente ausgeübt wird.

29. Vorrichtung gemäß Anspruch 1, wobei die Kontaktierungselemente austauschbar und ersetzbar sind.

30. Vorrichtung gemäß Anspruch 1 mit einem Taktgeber (140), der auf das Einschalten der Vorrichtung reagiert, zur Erzeugung fortlaufender Anzeigen von Intervallen der abgelaufenen Zeit, wobei die Intervalle der abgelaufenen Zeit zu den gewünschten Zeiten für die Behandlung von Gesichtsabschnitten in Beziehung stehen, wobei die fortlaufenden Anzeigen der Zeit in einer vorherbestimmten festen Zeitspanne bereitgestellt werden, während der die Vorrichtung in Betrieb ist; und Mittel zum Aufrechthalten des Status des Taktgebers für eine ausgewählte Zeitspanne nachdem die Vorrichtung ausgeschaltet worden ist und während der vorherbestimmten Zeitspanne nach dem Einschalten der Vorrichtung.

31. Vorrichtung gemäß Anspruch 30, wobei die fortlaufenden Anzeigen der Zeit in einer vorgegebenen festen Zeitspanne bereitgestellt werden, während der die Vorrichtung in Betrieb ist.

32. Vorrichtung gemäß Anspruch 31, wobei die feste Zeitspanne annähernd eine Minute ist und wobei die ausgewählten Intervalle der Zeit annähernd 10 bis 20 Sekunden betragen.

33. Vorrichtung gemäß Anspruch 30, wobei die Anzeigen ausgewählt sind aus a) einem hörbaren Signal und b) einer feststellbaren Veränderung in der Bewegung des sich bewegenden Kontaktierungselements.

34. Vorrichtung gemäß Anspruch 30, wobei die Anzeigen sowohl ein hörbares Signal als auch eine feststellbare Veränderung in der Bewegung des sich bewegenden Kontaktierungselements umfassen.

35. Vorrichtung gemäß Anspruch 30, die Mittel (146) zum Ändern des Status des Taktgebers zwischen aktiviert und deaktiviert aufweist, nachdem die Vorrichtung eingeschaltet wurde.

36. Vorrichtung gemäß Anspruch 35, wobei das Änderungsmittel auf einen An/Aus-Schalter reagiert, auf den für eine ausgewählte Zeitspanne gedrückt wird.

37. Vorrichtung gemäß Anspruch 36, wobei ein erstes hörbares Signal anzeigt, dass der Taktgeber aktiviert worden ist und ein zweites hörbares Signal, welches sich von dem ersten hörbaren Signal unterscheidet, anzeigt, das der Taktgeber deaktiviert worden ist.

38. Vorrichtung gemäß Anspruch 9, wobei die Reihen der Borstenbündel mehrere gerade Reihen aufweisen, von denen sich abwechselnd eine (a) bewegt (80) und (b) eine in ihrer Position fixiert bleibt (82).

39. Vorrichtung gemäß Anspruch 38, die eine weitere Reihe mit fixierten Borstenbündeln (84) aufweist, welche die mehreren geraden Reihen umgibt.

40. Vorrichtung gemäß Anspruch 9, wobei die Reihen der Borstenbündel mehrere gerade Reihen (70, 72) umfassen, die sich abwechselnd in entgegengesetzte Richtungen bewegen.

41. Vorrichtung gemäß Anspruch 40, die eine weitere Reihe mit fixierten Borstenbündeln (84) aufweist, welche die mehreren geraden Reihen umgibt.

42. Vorrichtung gemäß Anspruch 9, wobei die Reihen der Borstenbündel mehrere kreisförmige Reihen aufweisen, von denen sich abwechselnd eine (a) bewegt (90) und (b) eine in ihrer Position fixiert bleibt (92).

43. Vorrichtung gemäß Anspruch 42, die eine weitere Reihe mit fixierten Borstenbündeln (104) aufweist, welche die mehreren kreisrunden Reihen umgibt.

44. Vorrichtung gemäß Anspruch 9, wobei die Reihen der Borstenbündel mehrere kreisrunde Reihen (100, 102) umfassen, die sich abwechselnd in entgegengesetzte Richtungen bewegen.

45. Vorrichtung gemäß Anspruch 44, die eine weitere Reihe mit Borstenbündeln (104) aufweist, welche die mehreren kreisrunden Reihen umgibt.

## Revendications

1. Appareil pour le traitement de l'acné, comprenant :
au moins deux éléments de contact (57, 59) ayant des faces d'extrémité, au moins un élément de contact étant un élément de contact mobile (59) ;
un ensemble de montage (58, 60) pour maintenir les éléments de contact essentiellement adjacents l'un à l'autre ; et
un ensemble (50) pour bouger dans un mouvement de va-et-vient ledit au moins un élément de contact mobile ;
**caractérisé en ce que** toutes les faces d'extrémité sont dans le même plan et les faces d'extrémité de l'élément de contact mobile (59) bougent dans un seul plan ; et ledit ensemble bouge dans un mouvement de va-et-vient ledit au moins un élément de contact mobile de manière bidirectionnelle à travers une position neutre relativement à au moins un élément de contact adjacent, afin de produire une tension et une compression alternées de la peau, dans lequel, lorsque l'appareil est positionné de manière à ce que les faces d'extrémité des éléments de contact entrent en contact avec la peau, une action sur la peau dans le plan d'une zone de peau à traiter pour l'acné est produite pour enlever les bouchons de sébum des pores de la peau, permettant de les enlever facilement de la peau.

2. Appareil selon la revendication 1, dans lequel la fréquence de mouvement de l'élément de contact mobile est située dans une plage allant de 20 Hz à 1 kHz.

3. Appareil selon la revendication 2, dans lequel la fréquence est située de préférence dans la plage allant de 80 à 200 Hz.

4. Appareil selon la revendication 1, dans lequel les éléments de contact comprennent un matériau rigide.

5. Appareil selon la revendication 4, dans lequel les éléments de contact ont une surface de contact avec une rugosité située dans une plage allant de 0,127 µm à 0,508 µm (5 à 20 micropouces).

6. Appareil selon la revendication 4, dans lequel la rugosité est environ 0,254 µm (10 micropouces).

7. Appareil selon la revendication 4, dans lequel le rapport de l'amplitude maximum de mouvement de l'élément de contact mobile est situé dans une plage de 15 % à 120 % de la distance entre les éléments de contact adjacents bougeant l'un par rapport à l'autre.

8. Appareil selon la revendication 7, dans lequel ledit rapport est environ 60 %.

9. Appareil selon la revendication 1, dans lequel les éléments de contact comprennent, respectivement, des rangées (70, 72, 80, 82, 90, 92, 100, 102) de touffes de poils.

10. Appareil selon la revendication 9, dans lequel les poils ont un diamètre compris dans la plage allant de 0,0508 à 0,127 mm (2 à 5 millièmes de pouces) et une longueur comprise dans la plage allant de 6,35 à 15,24 mm (0,250 à 0,600 pouces), et dans lequel le matériau des poils a un module d'élasticité en flexion compris dans la plage allant de 689 MPa à 6,89 kPa (100 à 1000 kpsi).

11. Appareil selon la revendication 9, dans lequel les touffes ont des caractéristiques mécaniques telles qu'un déplacement de 1,58 mm (0,062 pouces) à la base d'une touffe produit une force comprise dans la plage allant de 0,3 grammes à 1,4 grammes, à exercer sur la peau à l'extrémité de ladite touffe.

12. Appareil selon la revendication 11, dans lequel la force est environ 0,61 grammes.

13. Appareil selon la revendication 9, dans lequel l'amplitude maximum de mouvement à la base d'une touffe de poils par rapport à celle d'un élément de contact adjacent est comprise dans la plage allant de 10 % à 125 % de la distance entre-axes entre des éléments de contact adjacents bougeant l'un par rapport à l'autre.

14. Appareil selon la revendication 13, dans lequel ladite amplitude maximum de mouvement à la base d'une touffe de poils (70) par rapport à celle d'un élément adjacent (72) est environ 45 %.

15. Appareil selon la revendication 1, dans lequel les éléments de contact comprennent un matériau souple.

16. Appareil selon la revendication 15, dans lequel les éléments de contact comprennent un matériau solide élastomère.

17. Appareil selon la revendication 15, dans lequel les éléments de contact comprennent un matériau en mousse.

18. Appareil selon la revendication 1, dans lequel le mouvement de va-et-vient de l'élément de contact mobile met la peau alternativement sous tension et sous compression.

19. Appareil selon la revendication 18, dans lequel l'amplitude maximum de mouvement de n'importe quel élément de contact mobile est un déplacement linéaire par rapport à un point sur un élément de contact adjacent, et dans lequel ledit déplacement linéaire est situé dans la plage allant de 10 % à 60 % de la distance entre les éléments de contact adjacents lorsqu'ils sont dans leur position neutre.

20. Appareil selon la revendication 19, dans lequel ledit déplacement linéaire est environ 40 %.

21. Appareil selon la revendication 18, dans lequel les éléments de contact sont composés d'un matériau rigide.

22. Appareil selon la revendication 21, dans lequel les éléments de contact ont un fini de surface compris dans la plage allant de 0,127 µm à 0,508 µm (5 à 20 micropouces) .

23. Appareil selon la revendication 22, dans lequel le fini de surface est environ 0,254 µm (10 micropouces).

24. Appareil selon la revendication 1, dans lequel le mouvement des éléments de contact mobiles est commandé par un moyen de commande (121, 122, 124), par quoi l'amplitude de mouvement des éléments de contact mobiles est sensiblement réduite lorsque les éléments de contact ne sont pas en contact avec la peau et par quoi l'amplitude de mouvement des éléments de contact mobiles est au niveau préféré lorsque les éléments de contact sont appliqués sur la peau avec une pression adéquate pour un fonctionnement optimal et par quoi l'amplitude de mouvement des éléments de contact mobiles est modifiée d'une manière détectable par l'utilisateur lorsque les éléments de contact sont appliqués sur la peau avec une pression plus grande que la pression adéquate.

25. Appareil selon la revendication 1, comprenant une structure d'alimentation en fluide (106) positionnée de façon adjacente aux éléments de contact et dans lequel ladite structure délivre un fluide qui entre en contact avec la peau pendant le fonctionnement.

26. Appareil selon la revendication 1, comprenant une structure contenant un fluide (106) positionnée de façon adjacente aux éléments de contact et dans lequel ladite structure contenant un fluide empêche essentiellement le fluide d'être expulsé de l'appareil par le mouvement des éléments de contact.

27. Appareil selon la revendication 26, dans lequel ladite structure contenant un fluide consiste en des touffes de poils ayant une extrémité libre à peu près dans le même plan que celui des éléments de contact.

28. Appareil selon la revendication 1, comprenant un ensemble de commande (121, 122, 124) pour contrôler la pression appliquée sur la peau de façon à minimiser la probabilité qu'une pression excessive de la peau soit exercée sur les éléments de contact mobiles.

29. Appareil selon la revendication 1, dans lequel lesdits éléments de contact sont amovibles et remplaçables.

30. Appareil selon la revendication 1, ayant un moyen de chronométrage (140), réceptif à la mise sous tension du dispositif, pour produire des indications successives d'intervalles de temps écoulé, ces intervalles de temps écoulé étant relatifs à des temps désirés pour traiter des parties du visage, les indications successives de temps étant fournies dans les limites d'une période de temps définie pré-établie pendant laquelle l'appareil fonctionne ; et un moyen pour maintenir l'état du moyen de chronométrage pendant une période de temps sélectionnée après que l'appareil a été mis hors tension et pendant ladite période de temps pré-établie après que l'appareil a été mis sous tension.

31. Appareil selon la revendication 30, dans lequel les indications successives de temps sont fournies dans les limites d'une période de temps définie pré-établie pendant laquelle l'appareil fonctionne.

32. Appareil selon la revendication 31, dans lequel la période de temps définie est environ une minute et dans lequel les intervalles de temps sélectionnés sont environ 10 à 20 secondes.

33. Appareil selon la revendication 30, dans lequel les indications sont sélectionnées entre a) soit un signal sonore, b) soit un changement détectable dans le mouvement de l'élément de contact mobile.

34. Appareil selon la revendication 30, dans lequel les indications comprennent à la fois un signal sonore et un changement détectable dans le mouvement de l'élément de contact mobile.

35. Appareil selon la revendication 30, comprenant un moyen (146) pour changer l'état du moyen de chronométrage entre activé et désactivé après que l'appareil a été mis sous tension.

36. Appareil selon la revendication 35, dans lequel le moyen de changement est réceptif à l'actionnement d'un interrupteur par pression pendant une période de temps sélectionnée.

37. Appareil selon la revendication 36, dans lequel un premier signal sonore indique que le moyen de chronométrage a été activé et un deuxième signal sonore différent du premier signal sonore indique que le moyen de chronométrage a été désactivé.

38. Appareil selon la revendication 9, dans lequel les rangées de touffes de poils comprennent une pluralité de rangées droites, avec des rangées alternées (a) qui bougent (80) et (b) qui restent fixes (82) en position.

39. Appareil selon la revendication 38, comprenant une autre rangée de touffes de poils fixes (84) entourant la pluralité de rangées droites.

40. Appareil selon la revendication 9, dans lequel les rangées de touffes de poils comprennent une pluralité de rangées droites (70, 72), avec des rangées alternées qui bougent dans des sens opposés.

41. Appareil selon la revendication 40, comprenant une autre rangée de touffes de poils fixes (84) entourant la pluralité de rangées droites.

42. Appareil selon la revendication 9, dans lequel les rangées de touffes de poils comprennent une pluralité de rangées circulaires, avec des rangées alternées (a) qui bougent (90) et (b) qui restent fixes (92) en position.

43. Appareil selon la revendication 42, comprenant une autre rangée de touffes de poils fixes (104) entourant la pluralité de rangées circulaires.

44. Appareil selon la revendication 9, dans lequel les rangées de touffes de poils comprennent une pluralité de rangées circulaires (100, 102), avec des rangées alternées qui bougent dans des sens opposés.

45. Appareil selon la revendication 44, comprenant une autre rangée de touffes de poils (104) entourant la pluralité de rangées circulaires.
